Europäisches Patentamt

European Patent Office

Office européen des brevets

(19)

(11) Veröffentlichungsnummer : **0 064 940**
**B1**

(12) **EUROPÄISCHE PATENTSCHRIFT**

(45) Veröffentlichungstag der Patentschrift :
04.05.88

(21) Anmeldenummer : **82730053.4**

(22) Anmeldetag : **04.05.82**

(51) Int. Cl.⁴ : **A 61 N   1/362**

(54) Herzschrittmacher.

(30) Priorität : 04.05.81 DE 3118097

(43) Veröffentlichungstag der Anmeldung :
17.11.82 Patentblatt 82/46

(45) Bekanntmachung des Hinweises auf die Patenterteilung : 04.05.88 Patentblatt 88/18

(84) Benannte Vertragsstaaten :
DE FR GB IT NL SE

(56) Entgegenhaltungen :
EP-A- 0 001 156
EP-A- 0 011 935
EP-A- 0 011 942
EP-A- 0 031 229
EP-A- 0 050 038
WO-A-79 /000 70
FR-A- 2 383 674
GB-A- 2 026 870
US-A- 4 181 133

(73) Patentinhaber : BIOTRONIK Mess- und Therapiegeräte GmbH & Co Ingenieurbüro Berlin
Sieversufer 8
D-1000 Berlin 47 (DE)

(72) Erfinder : Freiherr von Nettelhorst, Herwig, Dr.-Ing.
Pössnecker Strasse 30
D-1000 Berlin 45 (DE)
Erfinder : Schweiggert, Eugen
Selbitzer Strasse 81a
D-1000 Berlin 22 (DE)
Erfinder : Rexhausen, Hermann
Mellinger Strasse 48
D-3200 Hildesheim (DE)
Erfinder : Schaldach, Max, Prof. Dr.-Ing.
Turnstrasse 5
D-8520 Erlangen (DE)

(74) Vertreter : Christiansen, Henning, Dipl.-Ing.
CHRISTIANSEN & NINNEMANN Dietrich-Schäfer-Weg 21
D-1000 Berlin 41 (DE)

EP 0 064 940 B1

**Beschreibung**

Die Erfindung betrifft einen Herzschrittmacher der im Oberbegriff des Anspruchs 1 angegebenen Art. Aus der GB-A-2 026 870 ist ein derartiger künstlicher Herzschrittmacher bekannt, welcher durch vom Herzen aufgenommene Signale gesteuert wird, wie beispielsweise bei der durch Herzeigenaktionen getriggerten oder inhibierten Stimulation. Bei dem Einfall von Störsignalen bei dem betreffenden Eingang ist es erforderlich, daß der Schrittmacher einen Betriebszustand einnimmt, welcher ihn von den betreffenden Signalen unabhängig macht, da der fortgesetzte Einfall von Störsignalen mit einer gegenüber der Herzrate großen Frequenz im Falle von inhibierten Schrittmachersystemen die künstliche Stimulation unterbinden würde, auch wenn Herzeigenaktionen nicht tatsächlich vorhanden sind. Bei einer vom Eingangssignal getriggerten Stimulation wäre die Abgabe von Stimulationsimpulsen mit zu großer Frequenz die Folge. In beiden Fällen wäre der Patient in erheblichem Maße gefährdet, so daß der Übergang in einen Betrieb mit konstanter Stimulationsrate erforderlich ist.

Bei den bekannten Schrittmachern erfolgt der Übergang von einem Betriebszustand in den anderen zwangsläufig bei Auftreten von eine vorgegebenen Frequenz überschreitenden Eingangssignalen, welche als Störsignale sowohl innerhalb als auch außerhalb des Körpers des Patienten entstehen können und die Ursache beispielsweise in elektromagnetischen Feldern oder Elektrodenartefakten haben können. Bei den bekannten Herzschrittmachern erfolgt die Umschaltung der Betriebsart damit derart, daß nach der Implantation durch den behandelnden Arzt hier noch Einfluß genommen werden könnte. (In diesem Zusammenhang soll als Betriebsart — Mode — des Herzschrittmachers jeweils eine bestimmte Art und Weise der logischen Verknüpfung von Signalen innerhalb des Schrittmachers verstanden werden, welche zu Stimulationsimpulsen oder zur Unterdrückung von Stimulationsimpulsen führen).

Der Erfindung liegt die Aufgabe zugrunde, bei verändertem Krankheitsbild des Patienten eine Veränderung der Betriebsweise des Schrittmachers individuell gezielt vornehmen zu können, ohne daß die Neuimplantation eines anderen Typs erforderlich wäre.

Diese Aufgabe wird durch die im kennzeichnenden Teil des Anspruchs 1 angegebenen Merkmale gelöst.

Besonders vorteilhaft ist dabei, daß bei sich änderndem Herzverhalten des Patienten unverzüglich die Stimulationsart ohne operativen Eingriff durch Programmiermittel verändert werden kann, wobei weitere Umschaltungen vornehmbar sind, falls sich die erste der gewählten alternativen Stimulationsarten nicht als die geeignete erweist.

Dabei lassen sich noch weitere Betriebsarten, welche erst bei Störungen oder bestimmte Erscheinungsformen des Herzverhaltens hin wirksam werden sollen, vorprogrammieren und dabei insbesondere aus einer Mehrzahl von Betriebsartvarianten auswählen, so daß das Stimulationsverhalten auch bei Störfällen oder Tachykardiezuständen den augenblicklichen Bedürfnissen genau angepaßt werden kann.

Durch die zeitliche Synchronisation des Umschaltvorgangs mit dem Herz- bzw. Stimulationsverhalten des Schrittmachers ist sichergestellt, daß der Übergang von einer Betriebsart in die andere stets ohne Komplikationen und gefahrlos für den Patienten erfolgt.

Die Programmierungsmöglichkeit wird im Zusammenhang mit vorteilhaften Weiterbildungen, bei der unabhängig vom Herz oder Störungsverhalten unterschiedliche Alternativ-Betriebsweisen festlegbar sind, dadurch vereinfacht, daß mittels ebenfalls programmierbarer Schaltmittel für jede Art der bei der Änderung der Betriebsart zu berücksichtigenden Ereignisse aus einer Gruppe von Betriebszuständen ein erster, zweiter oder n-ter auswählbar ist. Die Zuordnung der sich alternativ einstellenden Betriebszustände zu den im Normalbetrieb wirksamen Betriebszuständen erfolgt durch eine Gruppe von Schaltmitteln matrixartig, wobei die Zuordnung, welcher der alternativen Betriebszustände jeweils wirksam wird, durch den gewählten Normalbetriebszustand bestimmt wird, von dem ausgegangen wird.

Bei diesen vereinfachten Programmierungsverfahren wird durch die vom Äußeren des Patienten zum inplantierten Schrittmacher hin zu übertragenden Signale einerseits bestimmt, welche Betriebsart im Normalbetrieb wirksam sein soll und dabei (gegebenenfalls über einen zusätzlichen Programmiervorgang anhand einer Tabelle für die jeweiligen Störungs- oder anormalen Zustände) ausgewählt, welcher der jeweils aufgeführten alternativen Betriebszustände im Störfall wirksam werden soll.

Bei einer Umprogrammierung der im Normalfall wirksamen Betriebsart werden dann automatisch die dieser Betriebsart durch die zuletzt programmierten Schalterstellungen zugeordneten Betriebsarten bei Eintreten der Zusatzbedingungen wirksam. Wenn — entsprechend einer anderen bevorzugten Ausführung der Erfindung diese alternativ einschaltbaren Betriebsarten funktionsbezogen einander zugeordnet sind, d. h. beispielsweise bei einer Störung in Atrium zwischen der alternativen Stimulation in einer Kammer oder Stimulation in beiden Kammern auswählen lassen, so ist eine derartige Zuordnung unabhängig von der gewählten Normalbetriebsart sinnvoll und braucht deshalb bei Wechsel der Normalbetriebsart durch Umprogrammierung bei ein und demselben Patienten vom Arzt in den seltensten Fällen zusätzlich verändert zu werden. Durch die gewählte Zuordnung ist also sichergestellt, daß der mit einer Umprogrammierung verbundene Handhabungs- bzw. Signalübertragungsaufwand ein Minimum ist. So kann beispielsweise durch entsprechende Verknüpfung der Schaltmittel festgelegt werden, daß bei Störsignalen im Atrium unter Unterbindung der Signalaufnahme aus dem Atrium im Falle des ursprünglichen AAI-Betriebs in den AAO-Betrieb übergegangen wird, während beim ursprünglichen VDT/I-Betrieb in den VVI-Mode übergegangen werden kann.

Mit dem programmierbaren Wechsel der Betriebsart in Abhängigkeit von den aufgefundenen irregulären Betriebszuständen des Schrittmachers lassen sich auch gleichzeitig vorzunehmende Wechsel einzelner oder mehrerer Betriebsparameter programmieren, wobei diese Parameter beispielsweise die Stimulationsfrequenz, deren Amplitude oder andere Zeitparameter betreffen. Bevorzugt wird beispielsweise beim Übergang in den Betriebszustand der AV-sequentiellen Stimulation im Falle von Tachykardie einer Verkürzung der AV-Überleitungszeit bei der Stimulation erzeugt, damit die Stimulation in die vulnerable Phase aufgrund unerwünschter Überleitungen verhindert ist.

Die Erfindung basiert auf dem Bestreben, eine universelle Schrittmacherstruktur anzugeben, welche Fehlermöglichkeiten bekannter Systeme vermeidet, wobei die Realisierung in einem implantierbaren System möglich sein soll. Das System soll eine weitestgehende Auswertung der aufgrund von vom Herzen abgeleiteten Signalen zur Verfügung stehender Informationen ermöglichen, wobei der Einfluß von Störsignalen gering gehalten ist. Durch die physiologisch korrekte Zuordnung aller die Betriebsarten des Schrittmachers betreffenden Umschaltvorgänge wird eine große Patientensicherheit erreicht.

Vorteilhafte Weiterbildungen sind in den Unteransprüchen angegeben und werden einschließlich der Erfindung anhand des in den Figuren dargestellten Ausführungsbeispiels näher erläutert. Es zeigen :

Figur 1 ein Blockschaltbild der den erfindungsgemäßen Schrittmacher bildenden Baugruppen unter besonderer Berücksichtigung der im Betrieb auftretenden logischen Verknüpfungen.

Figur 2 ein Zeitdiagramm zur Erläuterung des Funktionsablaufs bei dem in Fig. 1 dargestellten Schrittmacher sowie.

Figur 3 Einzelheiten einer zum Einschalten verschiedener Betriebszustände dienenden Baugruppe.

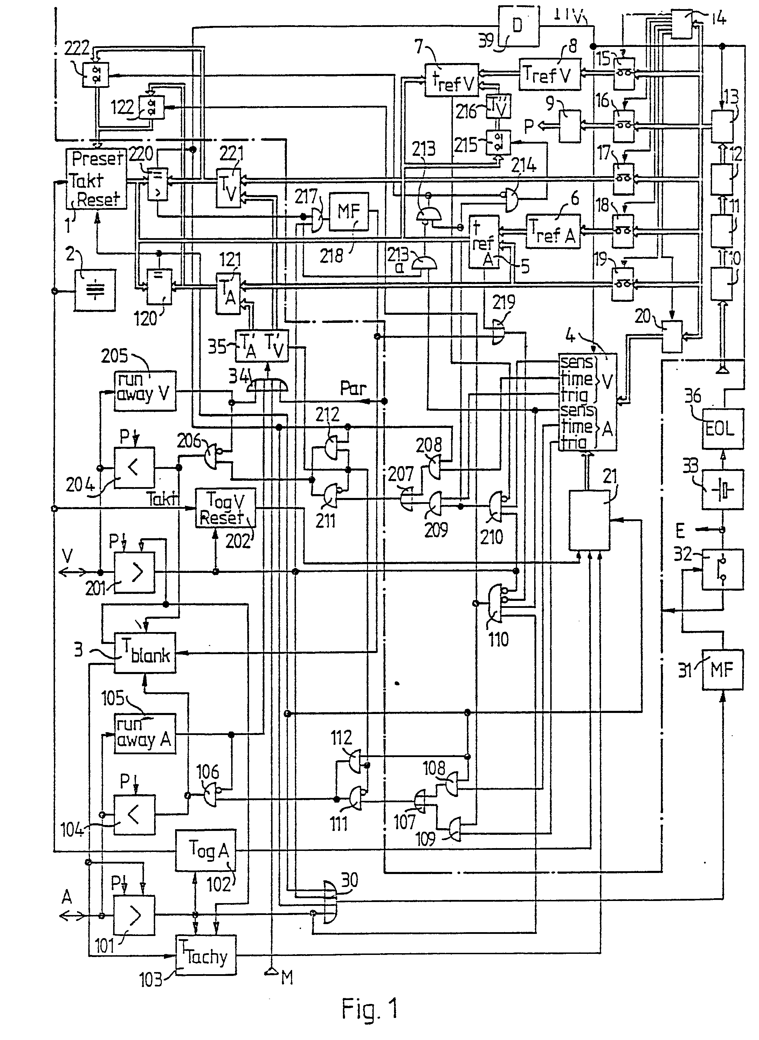

Das in Figur 1 dargestellte in Blockschaltungsform wiedergegebene Prinzipschaltbild des erfindungsgemäßen Herzschrittmachers gibt den Signalfluß zwischen den einzelnen Baugruppen und eine Anzahl von logischen Verknüpfungen wieder. Der verwendeten Schrittmacherstruktur liegt das Konzept eines Schrittmachers zugrunde, das auf grund von Zu- und Abschaltungen von Signalverbindungen in unterschiedlichen Betriebsarten (Modes) benutz werden kann, wobei die Umschaltung zwischen den einzelnen Betriebsarten (die im Folgenden mit den auf dem Gebiet der Herzschrittmachertechnik eingeführten generischen Codes bezeichnet werden sollen) programmiert und/oder signalabhängig erfolgen kann. In seinen komplexeren Modes paßt der Schrittmacher sich dabei auf physiologische Weise dem natürlichen Herzverhalten an.

Die Realisierung des dargestellten Konzepts kann gleichermaßen mittels diskreter oder programmierbarer Logik-Bauelemente erfolgen, wobei im Falle einer hardwaremäßigen Lösung die erforderlichen Verknüpfungen — wie dargestellt — mittels Logik-Gattern und Speichermitteln wie Flip-Flops und Latches erfolgen, während beispielsweise bei einer Mikroprozessorlösung die Logik-Entscheidungen nicht parallel, sondern nach Programm seriell vorgenommen werden. RAM-Speicherplätze übernehmen fabei die Funktion der Speichermittel zum Festhalten von für Betriebszustände charakteristischen Signalen. Einige der dargestellten Funktionsbaugruppen, stellen vorteilhafte Weiterbildungen der Erfindung dar.

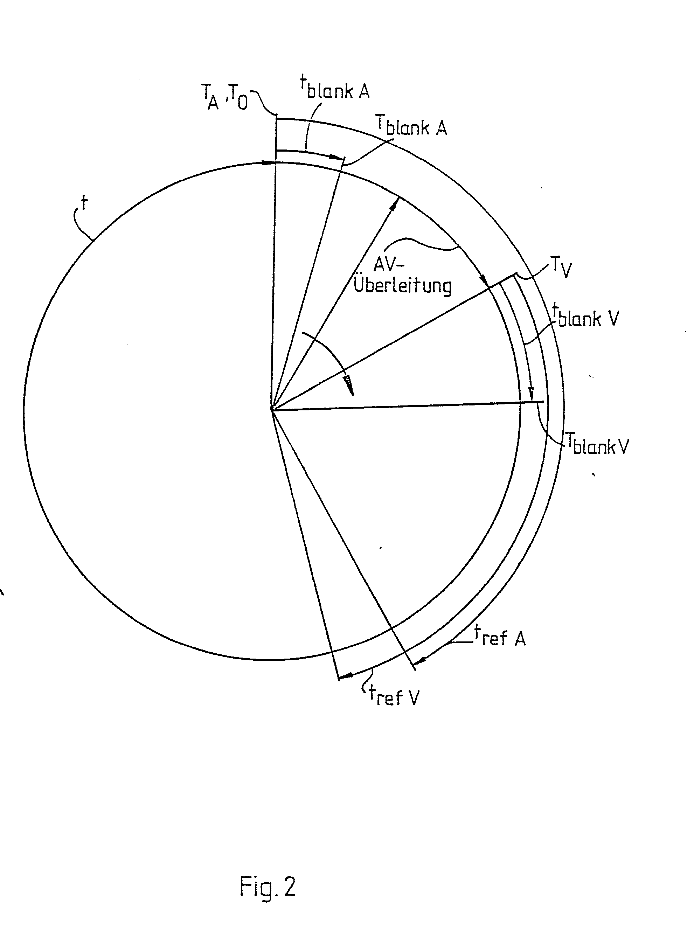

Aus Figur 2 ist der zeitliche Ablauf der Signalverarbeitung und Stimulationsvorgänge ersichtlich, wobei in den übrigen Darstellungen entsprechende Zeitbezeichnungen verwendet wurden, so daß diesbezüglich auf diese Figur stets Bezug genommen werden kann. In der Darstellung fällt der Anfangszeitpunkt $T_0$ zusammen mit dem Zeitpunkt $T_A$, der nach einem Umlauf (entsprechend einem Herzzyklus) erreicht wird und die Aktivität des Atriums charakterisiert. Im Falle einer Stimulation im Ventrikel oder Atrium folgt eine Austastzeit $t_{blankA}$ für die im Atrium plazierte Elektrode, wobei die Dauer dieser Austastzeit jeweils davon abhängig ist, ob im Atrium oder im Ventrikel, d. h. in derselben oder in der jeweils anderen Kammer stimuliert wurde. Das Ende dieser Austastzeit ist mit $T_{blankA}$ bezeichnet.

Zwischen $T_A$ und $T_V$ befindet sich die AV-Überleitungszeit, wobei $T_V$ den Zeitpunkt der Aktivität des Ventrikels charakterisiert. An $T_V$ schließt sich entsprechend eine Austastzeit $t_{blankV}$ an, welche im Zeitpunkt $T_{blankV}$ endet. Die Refraktärzeit des Atriums $t_{refA}$ beginnt mit $T_0$ und endet bei $T_{refA}$, während die Refraktärzeit des Ventrikels bei $T_V$ beginnt und bei $T_{refV}$ endet. Die Zeiten $T_{og}$ und $T_{tachy}$ sind aus dem Diagramm gemäß Figur 2 nicht direkt ersichtlich, da diese durch entsprechende Folgeraten der Herzaktionspotentiale und/oder Störsignale als Triggersignale festgelegt werden.

Bei dem in Figur 1 dargestellten Herzschrittmacher bilden die Anschlüsse A and V Ein- und Ausgänge für Signale, welche vom Herzen aufgenommen werden bzw. in Form von Stimulationsimpulsen an das Herz abgegeben werden. Mit « A » ist dabei der Anschluß für eine im Atrium und mit « V » der Anschluß für eine im Ventrikel fixierte Elektrode bezeichnet.

Die zentrale Steuerung für die zeitliche Zuordnung der abzugebenden Impulse erfolgt über einen Zähler 1, welcher durch von einem Taktgenerator 2 erzeugte Impulse inkrementiert wird und über einen « Reset »-Eingang in eine Ausgangsstellung zurücksetzbar ist. In Abhängigkeit von vom Herzen aufgenommenen Signalen ist dieser Zähler über einen zusätzlichen « Preset »-Eingang auf beliebige Zählerstände in Vorwärtsrichtung — entsprechend einer zeitlichen Voreilung — voreinstellbar. Ein Verändern des Zählerstandes in Rückwärtsrichtung ist nich notwendig. Nach Erreichen seines Endstandes setzt der nachfolgende Impuls den Zähler auf 0, von wo aus der Zählvorgang fortgesetzt wird. Das Zusammenwirken des Zählers mit den übrigen in Figur 1 dargestellten Bauelementen wird weiter unten näher beschrieben. (Die Übertragung einzelner digitaler Signalimpulse oder analoger Signale ist in den Figuren durch einfache Verbindungen dargestellt, während doppelte Linien die Übertragung vollständiger Datenwörter symbolisieren.)

Die vom Atrium über eine dort fixierte Elektrode aufgenommenen Signale gelangen über den Anschluß A an eine Eingangsstufe 101, welche einen Vorverstärker enthält, der über einen Programmiereingang (zugehöriger Pfeil P) in seinem Verstärkungsfaktor einstellbar ist. Die Eingangsstufe bewirkt gleichzeitig eine Impulsformung in der Weise, daß die vom Atrium aufgenommenen Signale in Form von impulsförmigen Logiksignalen in den nachfolgenden Schaltstufen weiterverarbeitet werden können. Die Darstellung der Logikpegel bei dem wiedergegebenen Ausführungsbeispiel erfolgt in positiver Logik, d. h. das Vorhandensein eines Signals wird mit dem H-Zustand und die Abwesenheit mit dem L-Zustand bezeichnet. Umschaltelemente (Zähler, Mono- und Flip-Flops) werden durch die ansteigenden Vorderflanken der Signalimpulse aktiviert. Weiterhin weist die Eingangsstufe 101 Filtermittel auf, mit einer Filtercharakteristik, die sie vorzugsweise für die aufzunehmenden Nutzsignale durchlässig macht.

Die Eingangsstufe 101 wird außerdem von einer Austastschaltung 3 beeinflußt, welche die Eingangsstufe für vorgegebene Zeiträume $t_{blankA}$ sperrt, wenn ein Stimulationsimpuls an Atrium oder Ventrikel abgeben wurde. Auf diese Weise wird verhindert, daß ein vom Schrittmacher abgegebener Stimulationsimpuls als herzeigenens Signal verarbeitet wird und das Betriebsverhalten des Schrittmachers fehlerhaft beeinflußt.

Der Eingangsstufe nachgeschaltet ist eine Störerkennungsschaltung 102, welche ein Ausgangssignal abgibt, wenn der Abstand zweier aufeinanderfolgender, von der Eingangsstufe 101 verarbeiteter Signalimpulse einen vorgegebenen zeitlichen Mindestabstand ($T_{og}$), entsprechend einer maximalen Folgefrequenz, unterschreitet. Derartige eine Störung bildende Signale können ohne Ursache. sowohl außerhalb (elektromagnetische Einstreuungen oder Wechselströme) als auch innerhalb des Körpers des Patienten (Elektrodenartefakte) haben. Die Störerkennungsschaltung 102 verhindert im Zusammenwirken mit weiter unten zu beschreibenden Mitteln, daß an der Atriumelektrode aufgenommene Impulse, welche die vorgegebene Frequenz überschreiten, zur Weiterverarbeitung zugelassen werden.

Der Ausgang der Eingangsstufe 101 ist weiterhin mit dem Eingang einer Tachykardieerkennungsstufe 103 verbunden, welche entsprechend ein Signal abgibt, wenn die am Ausgang der Stufe 101 erscheinenden Impulse eine vorgegebene Folgezeit unterschreiten. Die « Tachykardierate » $T_{Tachy}$ ist mit ca. 250 ms größer als die als Kriterium für das Vorhandensein eines Störsignals festgelegte Zeitdauer $T_{og}$, welche ca. 100 ms beträgt.

Der Anschluß V, an den eine im Ventrikel zu fixierende Elektrode angeschlossen werden kann, ist mit dem Eingang einer weiteren Eingangsstufe 201 verbunden, die in ihren grundsätzlichen Funktionen der Eingangsstufe 101 für vom Atrium · aufgenommene Signale entspricht. Der Verstärkungsfaktor ist über einen zusätzlichen Eingang (zugehöriger Pfeil P) einstellbar. Eine separate Störerkennungsschaltung 202 für die von der im Ventrikel fixierten Elektrode aufgenommenen Störsignale gibt ein Ausgangssignal ab, wenn die vom Ventrikel aufgenommenen Signalimpulse eine höhere als eine vorgegebene Frequenz aufweisen. Die Eingangsstufe 201 wird entsprechend durch von der Austaststufe 3 abgegebene Signale zeitweise gesperrt.

Stimulationsimpulse für das Atrium werden mittels einer Impulsformerschaltung 104 erzeugt, wobei die Amplitude der Stimulationsimpulse über einen zusätzlichen Eingang (zugehöriger Pfeil P) einstellbar ist. Die Impulsformerstufe 104 wird durch kurzzeitige Impulse mit logischem H-Pegel angesteuert und enthält auch die zur Heraufsetzung der Eingangsimpulse auf den zur Stimulation notwendigen Pegel erforderlichen Verstärkungsmittel.

Die von der Impulsformerstufe 104 abgegebenen Impulse gelangen einerseits zum Anschluß A und andererseits zu einer Run-away-Schutzschaltung 105, welche in dem dargestellten Ausführungsbeispiel durch ein Monoflop gebildet wird, dessen Impulsdauer auf die höchste zulässige Stimulationsrate im Atrium abgestimmt ist. Auf einen Ausgangsimpuls von der Impulsformerstufe 104 hin gibt die Schutzschaltung 105 einen Impuls vorgegebener Breite an den invertierenden Eingang eines UND-Gatters 106 ab, welches daraufhin für die Dauer des letztgenannten Impulses hin für an seinen anderen Eingang gelangende Signalimpulse gesperrt ist.

Eine Impulsformerstufe 204 für zum Stimulieren des Ventrikels bestimmte Impulse ist ebenfalls über einen weiteren Eingang (zugehöriger Pfeil P) bezüglich der Ampliude der abgegebenen Impulse beeinflußbar. Eine separate Run-away-Schutzschaltung 205 für den Ventrikel sperrt ein UND-Gatter 206 über dessen invertierenden Eingang, wenn die Folgezeit der den Ventrikel stimulierenden Impulse den vorgegebenen Wert unterschreitet.

Die Zeiten, zu denen Stimulationsimpulse abgegeben werden können (mit $T_A$ und $T_V$ bezeichnet), bestimmt der Zähler 1, wobei jeweils für Atrium und Ventrikel separate Vergleicherschaltungen 120 bzw. 220 vorgesehen sind, welche einen Impuls am Ausgang « = » erzeugen, wenn der Zählerstand des Zählers 1 mit einem vorgegebenen Zählerstand in einem Speicher 121 für den Stimulationszeitpunkt des Atriums bzw. einem entsprechenden Speicher 221 für den Ventrikel übereinstimmt. Die Vergleicherschaltung 220 gibt über einen zusätzlichen Ausgang « > » ein Signal ab, wenn der Zählerstand des Zählers größer ist als der in dem Speicher 221 festgehaltene Wert für $T_V$. Die genannten Zeitmarken $T_A$ und $T_V$ bilden Bezugszeiten für den Betrieb des Schrittmachers und beenden im Demand-Betrieb die sogenannten « Escape-Intervalle » innerhalb denen eine entsprechende Eigenaktion des Herzens die Erzeugung eines Stimulationsimpulses durch den Schrittmacher verhindert.

Die Zeiten $T_A$ und $T_V$ für Atrium und Ventrikel in den Speichern 121 bzw. 221 werden in Form von Zahlenwerten programmiert, welche diejenigen Zählerstände repräsentieren, die der Zähler 1 im Stimulationszeitpunkt erreicht haben muß. Die Zeitzuordnung zu den Zählerständen erfolgt in der Weise, daß die Stimulationszeitpunkte mit einem ausreichend feinen Zeitraster einstellbar sind. Bei einem Zählertakt von beispielsweise 1 kHz weist das zur Verfügung stehende Zeitraster eine Teilung in Millisekunden auf. Die mittels der Speicher 121 bzw. 221 vorgebbaren Zählerstände sind durch weiter unten darzustellende Mittel veränderbar.

Die Eingangsimpulse für die Ausgangsstufe 104, welche das UND-Gatter 106 passieren, stammen vom Ausgang eines ODER-Gatters 107, an dessen Eingänge alternativ die über die Abgabe eines Stimulationsimpulses an das Atrium entscheidenden Signale gelangen. Einer der Eingänge des ODER-Gatters 107 ist mit dem Ausgang eines UND-Gatters 108 verbunden, das ein Ausgangssignal abgibt, wenn die Vergleicherschaltung 120 das Erreichen des Zeitpunktes $T_A$ angibt und damit anzeigt, daß im Demand-Zustand der Vorhofstimulation ein Zeitpunkt erreicht wurde, bei dem wegen fehlender eigener Aktivität des Herzens ein Stimulationsimpuls erforderlich ist. Das UND-Gatter 108 wird über seinen weiteren Eingang mittels eines Signals durchgeschaltet, welches von einem Ausgang « time$_A$ » des Betriebszustandsregister 4 abgegeben wird, wenn eine Impulsabgabe nach Zeitablauf erwünscht ist. Bei freigegebener Signalaufnahme (sens$_V$ bei Block 4) für die entsprechende Kammer erfolgt damit ein « Demand »-Betrieb mit Inhibierung durch herzeigene Impulse, während bei gesperrter Signalaufnahme eine Stimulation zu festen Zeiten erfolgt.

Ein weiteres UND-Gatter 109 wird über einen entsprechenden Ausgang « trig$_A$ » der Schaltung 4 bei durch Herzeigenaktionen im Vorhof getriggerter Signalabgabe an den Vorhof aktiviert. Das Signal zum synchronen Auslösen des Stimulationsimpulses im Vorhof gelangt an den Eingang des UND-Gatters 109 vom Ausgang eines weiteren UND-Gatters 110, das seinerseits durch ein von einem Ausgang « sens$_A$ » der Schaltung 4 an seinen Eingang gelangendes Signal aktiviert wird, wenn die Aufnahme von Signalen aus dem Vorhof bei der gewählten Betriebsweise des Schrittmachers vorgesehen ist.

Es ist ersichtlich, daß für den durch herzeigene Signale getriggerten Betrieb diese Freigabe der Signalaufnahme Voraussetzung ist. An das UND-Gatter 110 gelangt nicht nur das Ausgangssignal der Eingangsstufe 101, sondern an dessen weiteren invertierenden Eingang auch das Ausgangssignal einer Refraktärstufe für den Vorhof 5, welche ein Signal abgibt, wenn für einen Zeitraum $t_{refA}$, der mit dem Erreichen des dem Zeitpunkt $T_A$ entsprechenden Zählerstand durch den Zähler 1 beginnt, die Verarbeitung der im Atrium aufgenommenen Signale zur Auswertung für die Abgabe von Stimulationsimpulsen gesperrt ist. Ein der Refraktärzeit $T_{refA}$ für das Atrium entsprechender Zahlenwert ist in einem Speicher 6 festgehalten und über weiter unten dargestellte Programmiermittel durch äußere Signale veränderbar. Ein vom Ausgang der Schaltung 201 im Falle der Signalaufnahme im Ventrikel gelangendes Signal sperrt über dessen weiteren invertierenden Eingang das UND-Gatter 110, so daß bei gleichzeitig im Vorhof und Ventrikel ankommenden Signalen, das Vorhofsignal gesperrt ist und somit der Ventrikel Priorität hat.

Ein ODER-Gatter 207 und UND-Gatter 208 bis 210 bilden die entsprechenden, über die Abgabe von Stimulationsimpulsen an den Ventrikel bzw. über die Signalaufnahme aus dem Ventrikel entscheidenden Logik-Gatter. Das gewünschte Betriebsverhalten des Schrittmachers läßt sich ebenfalls über drei Ausgänge « sens$_V$ », « time$_V$ » und « trig$_V$ » der Schaltung 4 bestimmen, wobei das an den weiteren Eingang des UND-Gatters 208 gelangende Signal die Stimulation freigibt, wenn der Stand des Zählers 1 dem im Speicher 221 festgehaltenen, dem Zeitpunkt $T_V$ zugeordneten Zählerstand erreicht und somit zu diesem Zeitpunkt ein Ausgangssignal des Vergleichers 220 an das UND-Gatter 208 ausgegeben wird.

Die synchrone Stimulation wird durch das UND-Gatter 209 ausgelöst, demdas Ausgangssignal des UND-Gatters 210, welches die vom Ventrikel aufgenommenen Signalimpulse weiterleitet, zugeführt wird. Das UND-Gatter 210 ist über seinen invertierenden Eingang während der Ventrikel-Refraktärzeit $t_{refV}$ gesperrt, welche mittels eines Vergleichers 7 festgelegt wird, wobei der erreichte Zählwert des Zählers 1 mit dem in dem Speicher 8 festgehaltenen, die Zeitdauer $T_{refV}$ repräsentierenden Zählwert verglichen wird und zu dem Inhalt des Speichers 8 derjenige des Speichers 216, dessen Inhalt $T_V''$ im Normalfall der Zeit $T_V$ entspricht, hinzuaddiert wird. (Unterschiede zwischen der Funktion der Speicher 6 für die Refraktärzeit des Atriums und 7 für die Refraktärzeit des Ventrikels sind aus der folgenden Beschreibung ersichtlich.) Der Inhalt des Speichers 8 ist wie derjenige des Speichers 6 über äußere Programmiermittel veränderbar.

Die Ausgangssignale der UND-Gatter 110 bzw. 210 beeinflussen des weiteren Schalter 122 und 222, mittels denen der Zähler 1 über seinen preset-Eingang auf die in den Speichern 121 (für den Wert $T_A$) bzw. 221 (für den Wert $T_V$) vorliegen-

den Werte voreinstellbar ist. Damit wird der Zähler 1 beim Erscheinen eines intrakardialen Signals im Atrium auf den der Zeit $T_A$ bzw. beim Erscheinen eines Signals im Ventrikel auf den der Zeit $T_V$ entsprechenden Zahlenwert vorangesetzt. Auf diese Weise wird der Schrittmacher mit dem Herzverhalten synchronisiert, wenn die UND-Gatter 110 bzw. 210 über ihre entsprechenden Signaleingänge für die Signalaufnahme vom Herzen freigegeben sind. Sind die zuletzt genannten Gatter gesperrt, so erfolgt die Stimulation gegebenenfalls asynchron.

Die Inhalte der Speicher 4, 6 und 8 sowie der eines weiteren Speichers 9, welcher die programmierbaren Verstärkungsfaktoren bzw. Ausgangssignalamplituden repräsentierenden Daten für die Stufen 101 und 201 sowie 104 und 204 (zugeordnete Pfeile P) enthält, sind über die blockschaltungsmäßig dargestellten Programmiermittel zu beeinflussen, welche nachfolgend kurz beschrieben werden sollen :

Die von außerhalb des Schrittmachers über einen geeigneten Nachrichtensignalträger (radiofrequente, optische oder sonstige Signale) in Form von Impulsen aufmodulierten, von einem entsprechenden — nicht dargestellten — Sender abgegebenen Signale, gelangen zu einem Empfänger 10, der die verstärkten Signale einem Dekoder 11 zuleitet, mit dessen Hilfe die zu übertragene ursprüngliche Programmierimpulsfolge wieder hergestellt wird. In einer Prüfstufe 12 wird mittels zusätzlich übertragener redundanter Signale festgestellt, ob die übertragene Information vollständig ist und letztere zutreffendenfalls an einen Steuerbaustein 13 übermittelt, durch den mittels einer ersten Teilinformation eine Auswahlschaltung 14 entsprechend aktiviert wird.

Durch das Ausgangssignal der Schaltung 14 wird eine der Schaltstufen 15 bis 20 betätigt, so daß die zweite übertragene Teilinformation in den mit der aktivierten Stufe der Stufen 15 bis 20 verbundenen Speicher gelangt und dort festgehalten wird. Die bereits erwähnten Speicherstufen 4, 6, 8, 9 sowie 121 und 222 halten die für den Betrieb des Schrittmachers wesentlichen veränderbaren Daten fest, wie sie in der vorangehenden Beschreibung aufgeführt wurden.

Der Speicher 4 ist von zusätzlichen Signalen beeinflußbar, welche aus der Signalverarbeitungsstufe 21 stammen, und eine Umschaltung der Betriebszustände in Abhängigkeit von vom Schrittmacher selbst aus dem Herzen aufgenommenen Signalen bewirkt, wobei die Eingangssignale der Stufe 21 von den Stufen 102 und 202 stammen und im Atrium bzw. im Ventrikel aufgenommene Störsignale repräsentieren, sowie von der Tachykardiestufe 103, welche ein Ausgangssignal abgibt, wenn der Tachykardiezustand festgestellt wurde.

Die von der strichpunktierten Linie umgebenen Bauelemente zur Signalverarbeitung und -speicherung sind jeweils nur zeitweise aktivert, da die zum Betrieb des Schrittmachers notwendigen logischen Operationen mittels moderner mikroelektronischer Bauelemente in sehr kurzen Zeiten ausgeführt werden können, so daß deren permanenter Betrieb einen unnötigen Energieverbrauch zur Folge haben würde.

Die außerhalb der genannten Linie befindlichen Bauelemente, die entweder direkt mit den im Herzen fixierten Elektroden verbunden sind und damit die Signalaufnahme vom Herzen kontrollieren, bzw. diejenigen Mittel, welche die Zeitgeber- und Zeitvergleichermittel bilden, sind dauernd aktiviert, da durch letztere bestimmt wird, zu welchen Zeitpunkten logische Operationen auszuführen sind.

Die Aktivierung der innerhalb der strichpunktierten Linie befindlichen Bauelemente erfolgt durch die von den Vergleichermitteln 120 bzw. 220 abgegebenen Signale, wenn ein vorgegebener Zeitpunkt erreicht wurde, oder aber durch die Ausgangssignale der Eingangsstufen 101 bzw. 201, wenn nämlich vom Herzen (außerhalb der jeweiligen Refraktärzeit) ein Signal aufgenommen wurde. Die Zusammenfassung der genannten Signale erfolgt über ein ODER-Gatter 30, welches über ein Monoflop 31 einen Schalter 32 betätigt, der die in der gewählten Darstellung innerhalb der strichpunktierten Linie angeordneten Bauelemente mit einer Energiequelle 33 verbindet, wobei dieses « Verbinden » mit einer Energiequelle in jedem Heraufsetzen der Energieversorgung, also auch in einem Umschalten der betreffenden Bauelemente von einem Bereitschafts-(Stand-by-) Zustand in einen Betriebszustand bestehen kann.

Die Impulsdauer des Monoflops 31 ist dabei so bemessen, daß zum Ausführen der notwendigen Schaltvorgänge ein ausreichend langer Zeitraum zur Verfügung steht. Diskrete Logikbauelemente lassen sich energiesparend bei derart herabgesetzter Versorgungsspannung betreiben, daß zwar keine Veränderungen vorgenommen werden können, gespeicherte Signalzustände jedoch erhalten bleiben. Diese Bauelemente dienen der nachfolgenden Signalverarbeitung in dem Sinne, daß sie festlegen, ob auf ein Eingangssignal oder beim Erreichen eines vorgegebenen Zeitpunkts ein Stimulationsimpuls abgegeben werden soll.

Der von der Energiequelle 33, die im dargestellten Ausführungsbeispiel von einer Batterie gebildet wird, ausgehende Pfeil E deutet an, daß die außerhalb der strichpunktierten Linie angeordneten Bauelemente direkt von der Energiequelle 33 ohne Zwischenschaltung des Schaltelementes 32 versorgt werden.

Mit der dargestellten Anordnung sind die Betriebsfunktionen üblicher Schrittmachertypen mit synchroner oder asynchroner Stimulation im Atrium und/oder Ventrikel erzeugbar, wobei je nach Zustand der Schaltmittel 4 und Freigabe der entsprechenden Stimulationssignale bzw. Signalaufnahmemöglichkeit über die UND-Gatter 108 bis 110 bzw. 208 bis 210 auch jene Schrittmacherbetriebsarten höherer Ordnung möglich sind, wie sie in Form einer Übersicht beispielsweise in dem Aufsatz « Physiological Cardiac Pacing », R. Sutton, J. Perrins und P. Citron in « PACE » ; Vol. 3, März-April 1980, S. 207 bis 219 wiedergegeben sind.

Das grundsätzliche Stimulationsverhalten der verschiedenen Schrittmacherbetriebsweisen bei verschiedenartigen Herzsignalen its ebenfalls in der vorgenannten Literaturstelle angegeben.

Weitere Einzelheiten zu den in der nachfolgenden Beschreibung dargestellten Schaltungsteilen können den am gleichen Tage eingereichten, diese Schaltungsteile wegen der damit verbundenen Neuerungen näher beschreibenden Patentanmeldungen WO 82/3780 bis 3786 derselben Anmelderin entnommen werden.

Durch die funktionsmäßige Trennung des komplexen, nur zeitweise aktivierten Logiksystems von den permanent betriebsbereiten Bauelementen, ist es in vorteilhafter Weise möglich, für den Fall des Ausfalls des umfangreichen Logikteils ein einfaches Ersatzsystem vorzusehen, durch welches ein Notbetrieb aufrecht erhalten werden kann. Obgleich zwar einerseits die Sicherheit einzelner Bauelemente gegen Ausfall in der Vergangenheit wesentlich gesteigert werden konnte, wird andererseits angestrebt, die Informationsverarbeitung in einem künstlichen Herzschrittmacher möglichst allen therapeutischen Anforderungen gerecht werden zu lassen, um dem Patienten eine optimale Stimulationstherapie zukommen zu lassen, und damit die erreichte Erhöhung der Zuverlässigkeit zum Teil wieder kompensiert.

Das dargestellte Konzept gestattet es, die Vorteile eines einfachen, höchst betriebssicheren Systems mit einem solcher höherer Verarbeitungsfähigkeit derart zu verbinden, daß bei Ausfall des komplexen Datenverarbeitungssystems ein einfaches Ersatzsystem bereitsteht, welches in der Lage ist, die Grundfunktionen des Schrittmachers unter nahezu allen Umständen für einen nur durch die Betriebsdauer der Energiequellen begrenzten Zeitraum sicherzustellen.

Diese Ersatzschaltung kann prinzipiell durch verschiedenartige Signale aktiviert werden. Eines dieser Signale ist ein von der komplexen Logikschaltung ausgehendes Signal « Par », das bei einer mittels eines Mikroprozessors realisierten Logikschaltung dann erscheint, wenn innerhalb der durchgeführten Operationen ein Plausibilitätsfehler ermittelt wurde, wie er beispielsweise durch eine Paritätsüberprüfung mit Hilfe von redundanten Signalen in bekannter Weise erzeugt werden kann. Entsprechende Signale lassen sich auch aus mit diskreten Logikbauelementen arbeitenden Schaltungen gewinnen, wenn hier zusätzliche Elemente verwendet werden, welche unzulässige Betriebszustände, beispielsweise in Form von Signalen, erkennen, die bei korrekt arbeitenden Logikmitteln beispielsweise nicht gleichzeitig oder aber nicht mit einer oberhalb oder unterhalb einer vorgegebenen Grenzfrequenz liegenden Rate etc. erscheinen dürfen. Derartige ein Fehlverhalten aufzeigenden Signale werden auch von den Ausgängen der Run-away-Schutzschaltungen 105 und 205 gewonnen und über ein ODER-Gatter 34 zusammengefaßt. Bei der dargestellten. Anordnung kann es theoretisch in dem Fall zu einem Ansprechen der Run-away-Schutzschaltungen bei ordnungsgemäß arbeitenden Taktgenerator 2 kommen, wenn in die Speicher 121 oder 221 durch einen Fehler in dem durch die strichpunktierte Linie umrandeten Teil der Logikschaltungen fehlerhafte Zahlenwerte eingelesen wurden.

Den ODER-Gatter 34 wird ein weiteres Signal M zugeführt, welches von einem Reed-Schalter stammt, der in der Zeichnung nicht dargestellt ist. Auf diese Weise ist es möglich, mittels eines von außen einwirkenden Magnetfeldes den Ersatzbetriebszustand entsprechend der « Magnetfrequenz » der bekannten Schrittmacher einzustellen.

Der Ausgang des ODER-Gatters 34 aktiviert eine Speicherschaltung 35, welche Zeitpunkten $T_A'$ und $T_V'$ entsprechende Zahlenwerte enthält, die bei Aktivierung dieser Speicherschaltung 35 mit Vorrang in die Speicher 121 bzw. 221 eingelesen werden. Gleichzeitig wird von der Schaltung 35 ein Ausgangssignal abgegeben, welches über invertierende Eingänge von UND-Gattern 111 und 211, die zwischen die Gatter 107 und 106 bzw. 207 und 206 eingeschaltet sind und diesen Signalweg sperren, während über die somit aktivierten UND-Gatter 112 und 212 eine direkte Verbindung zu den Ausgängen der Vergleicher 120 und 220 zergestellt wird.

Damit ist das im « D00 »-Betrieb arbeitende Ersatzsystem nicht mehr von der Signalverarbeitung der innerhalb der strichpunktierten Linie befindlichen Elemente der Schalzung abhängig. Die Werte $T_A'$ und $T_V'$ werden dabei so gewählt, daß sich eine für die Frequenzen annehmbare Ersatz-Stimulationsfrequenz ergibt. Die Betriebsart « D00 » bezieht sich auf den Anschluß zweier Elektroden. Ist jeweils nur eine der beiden Elektroden angeschlossen, so arbeitet der Schrittmacher im Zustand « A00 » oder « V00 » und gibt damit entsprechend der gewählten Elektrodenkonfiguration ebenfalls die zur Aufrechterhaltung der vitalen Funktionen notwendigen Stimulationsimpulse ab.

Mittels zweier in die Aktivierungsleitung des Schalters 222 eingefüger UND-Gatter 213 und 213a wird eine Synchronisation des Zählers 1 (und damit der Zeitsteuerung des Schrittmachers) auf vom Ventrikel erscheinende Signale für die Zeit der Überleitung vom Atrium zum Ventrikel verhindert (AV-Überleitung zwischen $T_A$ und $T_V$ in Figur 2), wenn gleichzeitig eine Signalaufnahme im Atrium möglich ist. Die entsprechenden Zeitmarken beziehen sich (abhängig von der Betriebsart des Schrittmachers) auf Zeiträume zwischen herzeigenen oder stimulierten Signalen. Dem UND-Gatter 213a werden das Signal $sens_A$ des Speichers 4 und das Signal « > » des Vergleichers 220 an seinen Eingängen zugeführt. Das Ausgangssignal des UND-Gatters 213a sperrt gegebenenfalls das UND-Gatter 213 über dessen invertierenden Eingang. Der weitere Eingang des UND-Gatters ist mit den die Signalaufnahme für das Atrium am UND-Gatter 110 freigebenden Signalausgang des Speichers 4 verbunden. Damit wird erreicht, daß eine Synchronisation bei regulärer Ventrikeltätigkeit stets nur mit $T_A$ erfolgt

und somit das Atrium bei AV-sequentieller Stimulation die Führung behält bzw. bei fehlender Signalaufnahme aus dem Atrium der Abstand aufeinanderfolgender Zeitpunkte $T_A$ konstant bleibt.

Der hier beschriebene Schrittmacher stellt damit die physiologisch richtige Synchronisation mit dem Atrium sicher, wenn dort ungestörte Signale zur Verfügung stehen. Ist die Signalaufnahme in Atrium gestört, so erfolgt — entsprechend den vorgesehenen Möglichkeiten der Betriebszustandsänderungen, die weiter unten beschrieben werden — eine Umschaltung in einen eine Signalaufnahme im Ventrikel ermöglichenden Betriebszustand, so daß auch in diesem Fall eine korrekte richtige Stimulation gewährleistet ist.

Obgleich bei freigegebener Signalaufnahme im Atrium für die Zeit t zwischen $T_A$ und $T_V$ keine Synchronisation des Zählers 1 durch im Ventrikel aufgenommene Signale erfolgt, wird die Refraktärzeit des Ventrikels dadurch in physiologisch sinnvoller Weise gesetzt, daß diese sich trotz fehlender Synchronisation des Zählers 1 auf die Zeiten bezieht, zu denen die Herzaktion im Ventrikel festgestellt wurde, was bei dem dargestellten Ausführungsbeispiel dadurch realisiert ist, daß das Ausgangssignal des UND-Gatters 210 über ein UND-Gatter 214 und ein Schaltelement 215 einen Speicher 216 mit einem zu diesem Zeitpunkt im Zähler 1 enthaltenen Zahlenwert lädt, so daß dieser Zeitpunkt $T_V''$ als Bezugszeitpunkt jetzt für die Ermittlung der Refraktärzeit $T_{refV}$ durch den Vergleicher 7 dienen kann. Erfolgt eine Dignalaufnahme ausschließlich im Ventrikel, so ist eine Synchronisation des Zählers durch das Schaltelement 222 im Zeitpunkt $T_V$ freigegeben, wodurch mittels des UND-Gatters 214 in den Speicher 216 dann derjenige Zahlenwert für $T_V$ übertragen wird, welcher auch im Speicher 221 vorhanden ist.

Nach Ablauf der Refraktärzeit für aus dem Ventrikel aufgenommene Signale erscheinende Impulse, welche von Extrasystolen herrühren, synchronisieren den Zeitzähler 1 des Schrittmachers, was bei dem dargestellten Ausführungsbeispiel dadurch erreicht wird, daß der Zähler 1 durch die Schaltermittel 222 auf den Wert $T_V$, der im Speicher 221 vorhanden ist, gesetzt wird. Darüberhinaus muß auch sichergestellt sein, daß retrograd übergeleitete Erregungen nach Extrasystolen keine Synchronisation des Schrittmachers bewirken. Dies wird dadurch erreicht, daß nach Erkennen der Extrasystole (Ventrikelaktivität vor dem Zeitpunkt $T_0$) die Atriumrefraktärzeit $t_{refA}$ für einen Zyklus auf 400 ms gesetzt wird, so daß für einen ausreichend langen Zeitraum (entsprechend der retrograden Überleitungszeit) — ausgehend von der Zeitmarke $T_V$ das Atrium für eine Signalaufnahme gesperrt ist.

Die Sperrung derartiger retrograder Überleitungen ist deshalb von besonderer Beteudung, weil ein vom Schrittmacher auf das Signal im Atrium hin mit der vorgegebenen AV-Überleitungszeit abgegebener Stimulationsimpuls im Ventrikel eine Stimulation in der vulnerable Phase zur Folge hätte.

Um hier den notwendigen Schutz zu bewirken, werden unmittelbar auf eine Extrasystole hin die Signalaufnahmemittel für Vorhof und Ventrikel für einen vorgegebenen Zeitraum refraktär geschaltet. Und zwar wird bei einem Eingangsimpuls am UND-Gatter 217 vom Ausgang der Eingangsstufe 201 für den Ventrikel, der erscheint, wenn auch der Vergleicher 220 am Ausgang « > » ein Signal abgibt, das an den zweiten Eingang des UND-Gatters 217 gelangt, ein Monoflop 218 gestartet. Dieses Monoflop gibt daraufhin einen Augangsimpuls von ca. 400 ms Dauer ab, welcher an einen Eingang des ODER-Gatter 219 gelangt, dessen zweiter Eingang mit dem Ausgang des Vergleichers 5 für die Refraktärzeit $T_{refA}$ verbunden ist, und dessen Ausgangssignal zu einem der Eingänge des UND-Gatters 110 übertragen wird.

Auf diese Weise ist sichergestellt, daß bei jeder nach der Zeit $T_V$ festgestellten Kammerkontraktion die Refraktärzeit für das Atrium neu gestartet und für die Zeitdauer des von dem Monoflop 218 abgegebenen Impulses (vorzugsweise 400 ms) aufrechterhalten wird, so daß eine Synchronisation des Schrittmachers durch retrograde Überleitungen verhindert ist. (Wie weiter unten näher erläutert ist, sind für diesen Zeitraum auch die Stör- und Tachykardieerkennungsmittel gesperrt, da diese bei einer retrograden Überleitung nicht ansprechen sollten. Die erforderliche Signalverbindung wird durch die Leitung vom Ausgang des Monoflops 218 zum Block 3 gebildet.) Ein ODER-Gatter sperrt auch den Ventrikel, wobei bevorzugt mittels — nicht dargestellter Schaltmittel — statt der Impulszeit des Monoflops 218 auch die in Block 7 enthaltene Zeit heranziehbar ist.

Für den Fall, daß gleichzeitig Signale im Atrium und Ventrikel aufgenommen werden, hat der Ventrikel Vorrang. Damit ist gewährleistet, daß die Sicherheit gegen schädliche Stimulation aufgrund unerwünschter Überleitungen auch bei nicht eindeutiger Signalerkennung zunächst einmal gegeben ist.

Gemäß einer bevorzugten Ausführung der Erfindung werden alle Umschaltungen (Programmänderungen, Betriebsartänderungen) zu Zeitpunkten vorgenommen, welche den Betriebsablauf nicht stören, das sind solche Zeitpunkte, zu denen keine Stimulation bewirkt werden kann und auch kein Eingangssignal erwartet wird, dessen Signalaufnahme wiederum von den eingestellten Betriebsparametern abhängig sein könnte. Ein derartiger Zeitpunkt ist derjenige auf $T_V$ folgende Zeitbereich, in dem beide Eingangsstufen refraktär sind (vergleiche Fig. 2, aus der die Zeiten des Herzzyklus im Hinblick auf den Betrieb des Schrittmachers in ihrer grundsätzlichen Verteilung ersichtlich sind).

Der mit $T_V$ beginnende Zeitraum ermöglicht dabei die für den Betrieb des Schrittmachers notwendigen Umschaltungen in einer Weise, welche die physiologischen Randbedingungen der Stimulation am wenigsten beeinträchtigen, da Umschaltungen zu dieser Zeit in eine natürliche Ruheperiode des Herzens fallen. Im Zeitpunkt $T_V$ finden auch solche Überprüfungen statt, die regelmäßig durchgeführt werden müssen, wie die Bestimmung des Betriebszustands der Energiequelle (EOL-Block 36).

Der Zeitpunkt $T_V$ wird bei normalem Herzverhalten stets durchlaufen und wäre an sich ausreichend beispielsweise die Übernahme der geänderten Betriebsparameter (entsprechender Pfeil an Speicherblock 4) oder die Änderung der Programmierung aufgrund der von außen her übertragenen entsprechenden Signale zu diesem Zeitpunkt zu bewirken (Pfeil nach Block 13).

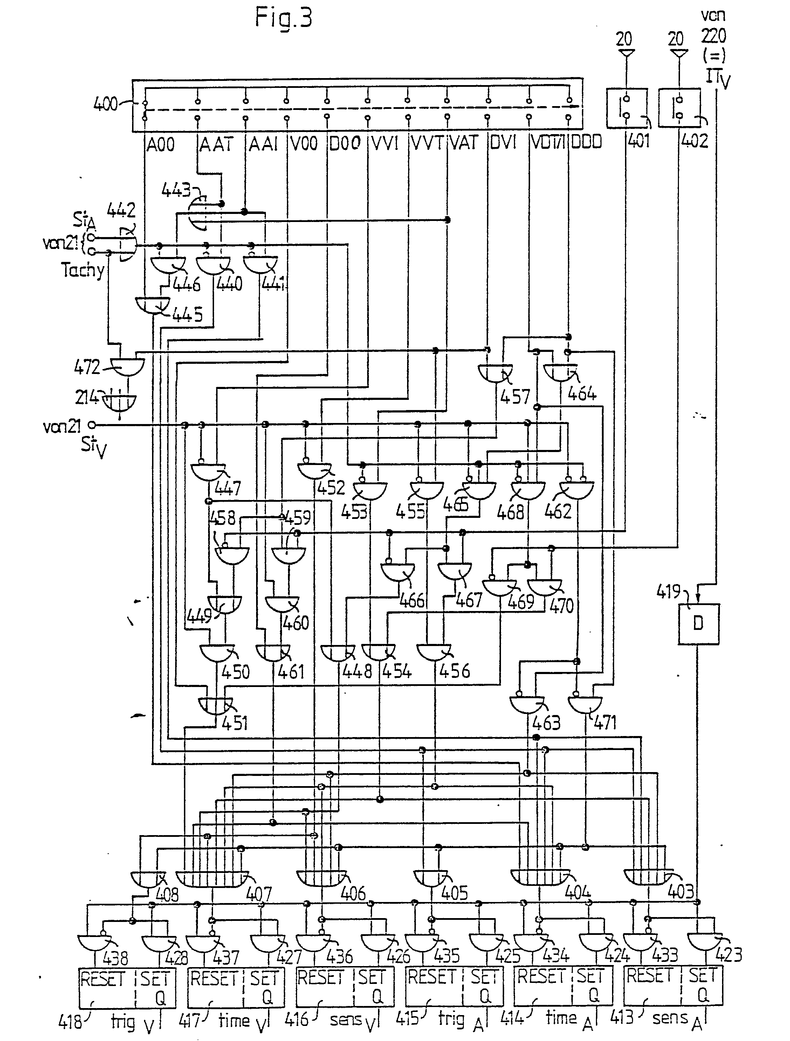

In Figur 3 sind diejenigen Bauelemente dargestellt, welche einerseits eine Programmierung vorgegebener Betriebsarten durch äußere Schaltmittel ermöglichen und weiterhin die Umschaltung der Betriebsart im Falle von an den Eingängen A und V einfallenden Störimpulsen bzw. im Falle einer Tachykardie im Vorhof bewirken. Insbesondere hervorzuheben ist dabei die Möglichkeit, den im Falle des Eintretens eines oder mehrerer der vorgenannten Ereignisse einzuschaltenden Betriebsart aus einer oder mehreren für diesen Fall festgelegten Betriebsarten auszuwählen und diese Auswahl vorhrer mittels Programmierung festzulegen. Bei der in Figur 3 dargestellten Schaltungsanordnung handelt es sich um diejenigen Baugruppen, die in der Blockschaltung gemäß Figur 1 als Steuerschaltung für die Betriebsarten in einem Element zusammengefaßt sind.

Durch das über das Schaltelement 20 zu dem Block 4 gelangende Programmiersignal wird einerseits mittels eines Schaltelementes 400 eine (permanente) Betriebsart des Schrittmachers ausgewählt, die dann gültig ist, wenn keine Störsignale aufgenommen werden und auch der Tachykardiezustand nicht vorhanden ist. Durch die Programmierung ist eine der Betriebsarten auswählbar. Zusätzlich kann — ebenfalls durch die vorgenommene Programmierung — durch weitere Schaltelemente 401 und 402 festgelegt werden, welcher von zwei möglicherweise zur Verfügung stehenden alternativen Betriebsarten im Falle des Auftretens einer oder mehrerer der genannten Bedingungen in Funktion treten soll, wobei beim Auftreten verschiedener Störungen der Schalter 401 bezüglich einer ersten Art und der Schalter 402 bezüglich einer zweiten Art von Störungen setzbar ist. Die bei dem dargestellten Ausführungsbeispiel gewählte Zuordnung kann je nach den gestellten Anforderungen geändert, eingeschränkt oder erweitert werden. So ist auch die Festlegung durch die Schalter 401 und 402 in der dargestellten Form nicht zwingend, sondern kann durch zusätzliche Schaltelemente in der Weise verändert werden, daß für unterschiedliche Störungs- oder Tachykardiezustände verschiedene alternative Betriebsfunktionen aktiviert werden.

Über die Ausgangsleitungen des Schaltelementes 400, die mit den üblichen Bezeichnungen für mögliche Schrittmacher-Betriebsfunktionen gekennzeichnet sind, werden (unter vorläufiger Außerachtlassung der für die Einschaltung der alternativen Betriebsfunktionen vorgesehenen Logik-Bauelemente) ODER-Gatter 403 bis 408 alternativ aktiviert, wobei jedem dieser ODER-Gatter 403 bis 408 ein Flip-Flop 413 bis 418 zugeordnet ist, an deren Ausgängen die das Betriebsverhalten des Schrittmachers bestimmenden Ausgangssignale, welche mit den Ausgangsleitungen des Blocks 4 in Figure 1 übereinstimmen, festgelegt werden.

Ein Setzen bzw. Zurücksetzen des Flip-Flops 413 im Falle einer Umprogrammierung durch das Signal $T_V$ erfolgt bevor eine Betriebsartänderung eintritt, wobei ein Verzögerungsglied 419 dafür sorgt, daß die Erkennung und Auswertung der die Betriebsart beeinflussenden Signale, die ebenfalls zum Zeitpunkt $T_V$ stattfindet, abgeschlossen ist.

Kriterium für die Bemessung der Verzögerungszeit des Gliedes 419 ist dabei, daß die Umschaltung der Betriebsart des Schrittmachers innerhalb derjenigen Zeit abgeschlossen sein muß, während der beide Eingänge A und V refraktär sind. (Es gilt auch für die übrigen von einem zum Zeitpunkt $T_V$ erscheinenden Impuls ausgelösten Signale, daß innerhalb des gesamten dargestellten Schrittmacherkonzepts — je nach Ausbauzustand — gegebenenfalls durch zusätzliche Verzögerung eine derartige Staffelung der Signalverarbeitung erfolgt, deß solche Schritte erst dann eingeleitet werden, wenn die vorangehenden Verarbeitungen, die für diese Schritte Voraussetzung sind, abgeschlossen sind. Es gilt dabei die Reihenfolge : Beendigung der Verarbeitung für den vorangegangenen Herzzyklus — Betriebsart-/Programmänderung — Festlegung der weiteren Daten für den nächsten Zyklus.)

Die Umschaltung der Flip-Flops erfolgt über UND-Gatter 423 bis 428 bzw. 433 bis 438, wobei je nach dem am Ausgang der ODER-Gatter 403 bis 408 anliegenden Signalzustände eines der beiden mit dem Setz- bzw. Rücksetzeingang der Flip-Flops 413 bis 418 verbundenen UND-Gatter aktiviert wird, wenn vom Ausgang der Schaltung 419 der gegenüber der Zeit $T_V$ verzögerte Impuls $I_{T_V}$ erscheint. Die Ausgänge der ODER-Gatter 403 bis 408 sind zu diesem Zweck jeweils mit einem nichtinvertierenden Eingang eines der UND-Gatter 423 bis 428 und einem invertierenden Eingang der UND-Gatter 433 bis 438 verbunden.

Die Funktionen der Flip-Flops 413 bis 418 sind die folgenden :

Flip-Flop 413 : Signalaufnahme in Atrium

Flip-Flop 414 : Zeitgesteuerte Auslösung von Stimulations impulsen im Atrium

Flip-Flop 415 : Durch herzeigene Signale getrigerte Auslösung von Stimulationsimpulsen im Atrium

Flip-Flop 416 : Signalaufnahme in Ventrikel

Flip-Flop 417 : Zeitgesteuerte Auslösung von Stimulationsimpulsen im Ventrikel

Flip-Flop 418 : Durch herzeigene Signale getrigerte Aus lösung von Stimulationsimpulsen im Ventrikel.

Es ist ersichtlich, daß aufgrund der bisher dargestellten Logikmittel in der Betriebsart « A00 » durch Setzen des Flip-Flops 414 das zeitgesteuerte Auslösen von Atriumimpulsen festgelegt ist. Eine Signalaufnahme aus Atrium oder Ventrikel erfolgt nicht und infolgedessen ist auch keine Umschaltung bei hier auftretenden Störsignalen erforderlich.

Entsprechendes gilt für die Betriebsarten « V00 » und « D00 », wobei hierbei noch das Flip-Flop 417 alternativ oder zusätzlich aktiviert ist.

Die Betriebsarten « AAT » und « AAI » aktivieren bei ungestörtem Atrium und, wenn keine Tachykardie vorhanden ist, über UND-Gatter 440 bzw. 441, deren invertierende Eingänge gemeinsam mit dem Ausgang eines ODER-Gatters 442 verbunden sind, an dessen Eingänge diese Zustände anzeigende Signale gelangen, wenn im Atrium eine Störung auftritt bzw. der Tachykardiezustand ermittelt wurde, die entsprechenden der Flip-Flops 413 bis 415. Ist am Ausgang des ODER-Gatters 442 ein Signal mit dem logischen H-Zustand vorhanden, so wird bei aktiviertem « AAT »- oder « AAI »-Zustand über ein ODER-Gatter 443 und ein UND-Gatter 446 sowie das ODER-Gatter 445 der « A00 »-Zustand eingestellt, wie er auch direkt durch eine entsprechende Programmierung gewählt werden kann.

Durch Aktivierung der Betriebsarten « V00 » bzw. « D00 » werden diese eingeschaltet, ohne daß eine Betriebsartsänderung durch über die Elektroden aufgenommenen Signale möglich wäre. Die Betriebsart « VVI » wird aktiviert, wenn dieser Zustand über den Programmschalter 400 gesetzt ist und keine Störungen im Ventrikel vorliegen, so daß ein Ausgangssignal vom UND-Gatter 447 an das ODER-Gatter 448 gelangt. Liegt eine Störung im Ventrikel vor, so geht der Schrittmacher durch die Verknüpfung der entsprechenden Signale über ein ODER-Gatter 449, ein UND-Gatter 450 sowie ein weiteres ODER-Gatter 451 in den Betriebszustand « V00 » über.

Die Betriebsart « VAT » ist bei ungestörtem Ventrikel (kein Signal am invertierenden Eingang des UND-Gatters 452) durch entsprechende Programmierung einstellbar.

In der Betriebsart « VAT » wird über das UND-Gatter 453 ein ODER-Gatter 454 aktiviert, wenn das UND-Gatter 453 nicht durch das Ausgangssignal des ODER-Gatters 442 über seinen invertierenden Eingang gesperrt ist. Im Falle einer Störung im Atrium oder des Vorliegens von Tachykardie erfolgt eine Umschaltung in den Zustand « V00 », was durch eine zum ODER-Gatter 443 führende Leitung bewirkt wird, wobei die Umschaltbedingungen denjenigen in den Modes « AAT » bzw. « AAI » entsprechen.

In der Betriebsart « DVI » wird bei ungestörtem Ventrikeleingang (kein Signal am invertierenden Eingang eines UND-Gatters 455), dessen Ausgangssignal zu einem ODER-Gatter 456 geleitet, welches die dieser Betriebsart entsprechenden Aktivierungen auslöst.

Bei gestörtem Ventrikel gelangt das Signal über ein ODER-Gatter 457 an die Eingänge zweier UND-Gatter 458 und 459, deren weitere Eingänge (wobei der weitere Eingang beim UND-Gatter 458 invertierend ist) mit dem Ausgang des Schalters 401 verbunden sind. Je nach dem, ob dieser durch Programmierung gesetzt (Verbindung mit logischem H-Pegel) oder nicht gesetzt (entsprechend dem logischen L-Pegel) ist, wird eines der UND-Gatter 458 bzw. 459 durchgeschaltet und aktiviert über das UND-Gatter 450 im Falle einer Störung im Ventrikel über das ODER-Gatter 451 den Zustand « V00 » oder über UND-Gatter 460, dessen weiterer Eingang entsprechend mit der bei gestörtem Ventrikel ein logisches « H-Signal » führenden Leitung verbunden ist, über ein ODER-Gatter 461 den Zustand « D00 ». Damit kann von außerhalb des Schrittmachers her für den Fall eines im Ventrikel aufgenommenen Störsignals durch eine entsprechende Voreinstellung festgelegt werden, welchen Betriebszustand der Schrittmacher in diesem Fall einnehmen wird. Somit besteht eine zusätzliche therapeutische Möglichkeit in der Art, daß der Arzt für den Fall, daß ein ursprünglich von ihm gewählter Betriebszustand des Schrittmachers nicht beibehalten werden kann, einen Alternativzustand auswählen kann, der sich im Fall des betreffenden Patienten als besonders günstig darstellt.

Eine entsprechende Wahlmöglichkeit ist auch beim Betriebszustand « VDT/I » vorgesehen, wobei das Fehlen von Störungen im Atrium und Venzrikel und auch das Nichtvorliegen des Tachykardie-Zustands mittels eines UND-Gatters 462 ermittelt wird, dessen beide invertierende Eingänge einerseits mit dem Ausgang des ODER-Gatters 442 bzw. mit der eine Störung im Ventrikel anzeigenden Leitung verbunden sind. Der Ausgang des UND-Gatters 462 führt zu einem UND-Gatter 463, dessen Ausgangssignal die für den Betriebszustand « VDT/I » notwendigen logischen Verknüpfungen auslöst. Im Falle einer Störung im Atrium allein wird über ein ODER-Gatter 464 und ein UND-Gatter 465 bei Vorliegen einer Störung im Atrium bzw. Tachykardie und Abwesenheit einer Störung im Ventrikel je nach Stellung des Schalters 401 über UND-Gatter 466 bzw. 467 entweder der Zustand « VVI » oder der Zustand « DVI » aktiviert.

Ist dagegen der Ventrikel gestört, während der Sinusrhythmus regulär aufgenommen wird, gelangt über ein diese Signalzustände auswertendes UND-Gatter 468 in Abhängigkeit von der Stellung des Schalters 402 ein Signal zu UND-Gattern 469 und 470 alternativ zu den ODER-Gattern 451 bzw. 454, so daß in diesem Fall alternativ die Betriebszustände « V00 » oder « VAT » aktivierbar sind. Bei gestörtem Ventrikeleingangssignal bleibt demnach dem behandelnden Arzt die Möglichkeit, entweder im Atrium auftretende Signale noch zur Triggerung der Ventrikel-Stimulation heranzuziehen oder aber den Ventrikel starrfrequent zu stimulieren. Der Betriebszustand « VDT/I » geht damit beispielsweise in den « VAT »-Zustand über.

Der Fall des ungestörten Betriebs im Mode « DDD » wird entsprechend der Funktion des UND-Gatters 463 für den Fall « VDT/I » über ein UND-Gatter 471 signalisiert.

Bei gestörtem Atriumeingang bzw. vorliegender Tachykardie werden die Umschaltungen entsprechend wie im vorgenannten Betriebszustand über das ODER-Gatter 464 ausgelöst, während bei gestörtem Ventrikel ebenfalls eine entsprechende Verknüpfung über das ODER-Gatter 457 herbeigeführt wird.

Es ist ersichtlich, daß die vorgenannten Mode-Umschaltungen und die Möglichkeit der Vorprogrammierung alternativer Modes im Falle von Störungen der Signaleingänge oder Tachykardie auf unterschiedliche Weise vorgegeben werden können, wobei insbesondere für die Terminierung von Tachykardien auch Sonderbetriebsweisen aktiviert werden können, die an dieser Stelle nicht detailliert beschrieben zu werden brauchen, da sie dem Fachmann aus entsprechenden Veröffentlichungen bekannt sind.

Die Möglichkeit, mit einer Mode-Änderung auch Betriebsparameter zu verändern, ist für den Fall des DVI-modes durch die Verbindung vom Ausgang « DVI » des Schalters 400 zu einem Eingang des ODER-Gatters 214 über ein UND-Gatter 472 wiedergegeben. Bei Aktivierung dieses UND-Gatters 472 im Falle von Tachycardie (Leitung zum Eingang « Tachy ») beim « DVI »-Betrieb erfolgt eine zwangsläufige Verkürzung der programmierten AV-Überleitungszeit ($T_0$ bis $T_V$) auf einen unkritischen Wert, der für diese Ausführung als in Block 216 enthalten angenommen werden soll.

**Patentansprüche**

1. Herzschrittmacher mit Mitteln zur Abgabe von Stimulationsimpulsen und Mitteln zur Veränderung der Betriebsart (mode) mit Mitteln (107 bis 110 und 207 bis 210), welche die für mindestens zwei Betriebsarten zu erzeugenden logischen Verknüpfungen von Signalen für die Übermittlung von Signalzuständen auf unterschiedliche Steuersignale hin aktivieren, wobei die Verknüpfung von Signalen und Betriebsarten durch Schaltmittel in der Weise vorgesehen ist, daß in Abhängigkeit von der gewählten Betriebsart die Übermittlung und/oder Verarbeitung verschiedener Ein- und Ausgangssignale oder Steuerzeiten repräsentierende Signale unterbunden bzw. zugelassen wird, die Steuersignale mittels externer Programmierungsmittel durch Fernsteuerung übertragbar sind, und Schaltmittel vorgesehen sind, welche in Abhängigkeit von vorgegebenen Signalzuständen im Herzen und in Abhängigkeit von einer Ausgangsbetriebsart, bei der dieser Signalzustand nicht vorliegt, und dem aufgefundenen Signalzustand die Umschaltung auf eine zugeordnete andere Betriebsart aktivieren, dadurch gekennzeichnet, daß die Zuordnung der in Abhängigkeit von vorgegebenen Signalzuständen im Herzen und der Ausgangsbetriebsart auszuführenden Umschaltungen ebenfalls mit Hilfe von Programmierungsmitteln (10 bis 13) durch Fernsteuerung veränderbar ist.

2. Herzschrittmacher nach Anspruch 1, dadurch gekennzeichnet, daß mit der Betriebsart neben zu schaltenden Verbindungen für die analoge und/oder digitale Übermittlung von Signalzuständen auch Betriebsparameter veränderbar sind, welche Steuerzeiten und/oder Signalamplituden betreffen.

3. Herzschrittmacher nach Anspruch 2, dadurch gekennzeichnet, daß für einen Betriebszustand mit Stimulation in Atrium und Ventrikel der programmierte zeitliche Abstand zwischen den Stimulationsimpulsen in Atrium und Ventrikel auf einen Wert von 100 bis 150 ms verkürzt wird.

4. Herzschrittmacher nach einem der vorangehenden Ansprüche, dadurch gekennzeichnet, daß es sich bei den Signalzuständen im Herzen um im Atrium und/oder Ventrikel aufgenommene Störsignale bzw. um den Zustand der Tachykardie kennzeichnende Signale handelt.

5. Herzschrittmacher nach einem der vorangehenden Ansprüche, dadurch gekennzeichnet, daß die Schaltmittel, welche in Abhängigkeit von vorgegebenen Signalzuständen im Herzen und in Abhängigkeit von der Ausgangsbetriebsart, bei der dieser Signalzustand nicht vorliegt, und dem aufgefundenen Signalzustand mindestens eine zugeordnete weitere Betriebsart aktivieren, wobei die Verknüpfung matrixartig in der Weise vorgesehen ist, daß die Ausgangsbetriebsarten die Spalten und die Signalzustände die Zeilen der Matrix, bzw. umgekehrt, bestimmen, während die mindestens eine weitere Betriebsart die Elemente der Matrix (Figur 3) bilden.

6. Herzschrittmacher nach einem der vorangehenden Ansprüche, dadurch gekennzeichnet, daß als Verbindungen die Signalaufnahmeverbindungen zu Atrium und/oder Ventrikel, die Signalabgabeverbindungen zur Stimulation von Atrium und/oder Ventrikel und/oder entsprechend die Stimulationsbedingungen, und damit die von aufgenommenen Signalen getriggerte und/oder inhibierte Stimulation jeweils separat abschaltbar ist.

7. Herzschrittmacher nach einem der vorangehenden Ansprüche, dadurch gekennzeichnet, daß die Umschaltmittel für die Betriebsart durch äußere Programmierungsmittel, auf die Aufnahme von Störsignalen und/oder vorgegebene Signalzustände in einer der oder beider Herzkammern und/oder eine Prüfschaltung für den Zustand einer Energiequelle (EOL) oder aber durch die Signalverarbeitungsmittel aktivierbar sind nach einer vom Ventrikel herrührenden Herzaktion bzw. nach Erreichen des Zeitpunkts, zu dem ein Stimulationsimpuls an den Ventrikel abzugeben ist, während der Signaleingang bzw. die Signaleingänge für vom Herzen abgeleitete Signale refraktär sind.

**Claims**

1. A heart pacemaker with means for emitting stimulation pulses and means for changing the mode of operation, with means (107 to 110 and 207 to 210), which activate the logic linkages of signals, which are to be procuded for at least two modes of operation, for transmitting signal conditions in response to different control signals, in which the linking of signals and modes of operation is provided by switching means in such a way that dependent on the mode of operation selected, the transmission and/or processing of different input and output signals, or signals representing control times, is prohibited or permitted, the control signals may be transmitted with the aid of external programming means by remote control and switching means are provided which, dependent on preset signal conditions in the heart and dependent on an initial mode of operation, in which this signal condition is not present, and on the signal condition as detected activate the switchover to an associated other mode of operation characterised in that the association of the switchovers, which are to be implemented dependent on preset signal conditions in the heart and on the initial mode of operation, may also be changed with the aid of programming means (10 to 13) by remote control.

2. A heart pacemaker according to claim 1, characterised in that, apart from connections which are to be switched for analog and/or digital passing on of signal conditions, operating parameters may also be changed with the mode of operation and relate to control times and/or signal amplitudes.

3. A heart pacemaker according to claim 2, characterised in that for an operating condition with stimulation in the atrium and ventricle the programmed time spacing between the stimulation pulses in the atrium and the ventricle is shortened to a value of 100 to 150 ms.

4. A heart pacemaker according to any one of the preceding claims, characterised in that the signal conditions in the heart relate to disruptive signals picked up in the atrium and/or ventricle or to signals characterising the condition of tachycardia.

5. A heart pacemaker according to any one of the preceding claims, characterised in that the switching means, which activate, dependent on preset signal conditions in the heart and on the initial mode of operation, in which this signal condition is not present, and on the signal condition as found, at least one associated further mode of operation, in which the linking is provided in matrix form in such a way that the initial modes of operation determine the columns and the signal conditions determine the rows of the matrix, or vice versa, while the at least one further mode of operation form the elements of the matrix (Figure 3).

6. A heart pacemaker according to any one of the preceding claims, characterised in that as connections the signal pick up connections at the atrium and/or ventricle, the signal emission connections for stimulating the atrium and/or ventricle and/or accordingly the stimulation requirements, and thus the stimulation inhibited and/or triggered by picked up signals may be switched off separately in each case.

7. A heart pacemaker according to any one of the preceding claims, characterised in that the switchover means for the mode of operation may be activated by external programming means, in response to pick up of disruptive signals and/or preset signal conditions in one or both of the heart chambers, and/or a test circuit for the condition of an energy source (EOL) may be activated alternatively by the signal processing means after action of the heart emanating from the ventricle or after reaching the point in time at which a stimulation pulse is emitted to the ventricle, while the signal input or the signal inputs are refractory for signals derived from the heart.

## Revendications

1. Stimulateur cardiaque comprenant des moyens pour délivrer des impulsions de stimulation et des moyens pour changer le mode de fonctionnement, avec des moyens (107 à 110 et 207 à 210) qui, sous l'effet de différents signaux de commande, produisent les combinaisons logiques de signaux à établir pour la transmission d'états de signal dans au moins deux modes, la combinaison de signaux et de modes étant réalisée par des moyens de commutation, de manière que la transmission et/ou le traitement de différents signaux d'entrée et de sortie, ou de signaux représentant des temps de commande, soit interdit ou autorisé suivant le mode choisi, les signaux de commande étant transmissibles par télécommande par des moyens de programmation externes et des moyens de commutation étant prévus pour produire la commutation sur un autre mode, coordonné, en fonction d'états de signal préfixés dans le cœur et en fonction d'un mode initial, avec lequel cet état de signal n'existe pas, et du mode constaté, caractérisé en ce que la coordination des commutations à effectuer, en fonction d'états de signal préfixés dans le cœur et du mode initial, peut également être changée par télécommande par des moyens de programmation (10 à 13).

2. Stimulateur selon la revendication 1, caractérisé en ce que, avec le mode de fonctionnement, outre des connexions à commuter pour la transmission analogique et/ou numérique d'états de signal, il permet aussi de changer des paramètres de fonctionnement qui concernent des temps de commande et/ou des amplitudes de signal.

3. Stimulateur selon la revendication 2, caractérisé en ce que, pour un mode de fonctionnement avec stimulation dans l'oreillette et le ventricule, l'intervalle temporel programmé entre les impulsions de stimulation dans l'oreillette et le ventricule est raccourci à 100-150 ms.

4. Stimulateur selon une des revendications précédentes, caractérisé en ce que les états de signal dans le cœur se rapportent à des signaux parasites recueillis dans l'oreillette et/ou dans le ventricule ou à l'état de signaux caractérisant la tachychardie.

5. Stimulateur selon une des revendications précédentes, caractérisé en ce que les moyens de commutation destinés à produire la commutation sur un autre mode, en fonction d'états de signal

préfixés dans le cœur et en fonction d'un mode initial, avec lequel cet état de signal n'existe pas, et du mode constaté, rendent opératoire au moins un mode coordonné supplémentaire, la combinaison étant réalisée, à la façon d'une matrice, de manière que les modes initiaux fixent les colonnes et les états de signal fixent les lignes de la matrice, ou inversement, tandis que le mode supplémentaire ou les modes supplémentaires forment les éléments de la matrice (figure 3).

6. Stimulateur selon une des revendications précédentes, caractérisé en ce que les connexions avec l'oreillette et/ou le ventricule pour recueillir des signaux, les liaisons pour délivrer des signaux en vue de la stimulation de l'oreillette et/ou du ventricule et/ou, de façon analogue, les conditions de stimulation et par suite la stimulation déclenchée et/ou inhibée par les signaux recueillis, constituent chaque fois des liaisons susceptibles d'être coupées séparément.

7. Stimulateur selon une des revendications précédentes, caractérisé en ce que les moyens de commutation pour le mode de fonctionnement peuvent être actionnés par des moyens de programmation externes, lorsque des signaux parasites et/ou des états de signal préfixés sont recueillis dans l'une ou les deux cavités du cœur, et/ou par un circuit de contrôle pour l'état d'une source d'énergie (EOL), ou encore par les moyens de traitement des signaux, à la suite d'une action cardiaque provenant du ventricule ou lorsque est atteint l'instant où une impulsion de stimulation doit être délivrée au ventricule, pendant que l'entrée de signal ou les entrées de signal pour les signaux tirés du cœur sont rendues inopérantes.

Fig. 1

Fig. 2

Fig.3